# FASCICULE DE BREVET EUROPEEN

(11) **EP 1 898 783 B1**
(45) Date de publication et mention de la délivrance du brevet: **12.03.2014**
(21) Numéro de dépôt: 06763845.2
(22) Date de dépôt: 22.06.2006
(51) Int. Cl.: A61B 5/05, A61B 5/00, A61B 5/053

(54) **SYSTEME ET PROCEDE D'ANALYSE ELECTROPHYSIOLOGIQUE**
ELEKTROPHYSIOLOGISCHES ANALYSESYSTEM UND VERFAHREN
ELECTROPHYSIOLOGICAL ANALYSIS SYSTEM AND METHOD

(30) Priorité: 22.06.2005 FR 0506321; 29.11.2005 FR 0512068; 13.02.2006 FR 0601239
(43) Date de publication de la demande: 19.03.2008
(73) Titulaire: Impeto Medical, 75014 Paris (FR)
(72) Inventeur: BRUNSWICK, Philippe, F-75014 Paris (FR); TOURNEFIER, Annick Nicole Lydie, 21610 Fontaine (FR); BOCQUET, Nicolas, 92310 Chatillon (FR)
(74) Mandataire: Regimbeau
(86) Numéro de dépôt international: PCT/EP2006/063452
(87) Numéro de publication internationale: WO 2006/136598

(56) Documents cités:
- EP-A2- 0 942 278
- WO-A-00/19894
- WO-A1-2006/044868
- US-A- 4 794 934
- US-A- 5 782 884
- US-A- 5 800 350
- US-A1- 2002 107 452
- US-A1- 2004 128 088
- US-A1- 2004 128 088
- US-A1- 2005 113 650
- US-B1- 6 512 949
- [en ligne] Extrait de l'Internet: <URL:http://web.archive.org/web/20050405232 628/http://en.wikipedia.org/wiki/Outlier>

## Description

La présente invention concerne d'une façon générale les appareils de diagnostic médical dans le domaine de la santé humaine ou animale.

Compte tenu du coût des analyses de sang et du caractère invasif des prélèvements sanguins préalables, les médecins sont aujourd'hui de plus en plus réticents à prescrire à leurs patients des bilans complets trop fréquents.

Il en résulte une évidente sous-détection de certaines maladies dont la détection s'effectue principalement par analyse de sang, telles que le diabète, l'hypertension, l'hyperthyroïdie, les maladies des coronaires, ....

De plus lorsque ces maladies sont détectées, il est souvent très difficile ou coûteux d'évaluer l'efficacité du traitement prescrit, car il est impossible dans la pratique d'effectuer des analyses biologiques journalières.

On connaît par ailleurs différents systèmes de mesure électrophysiologique tels que les appareils d'électrocardiographie et d'électroencéphalographie. Ces systèmes sont passifs en ce sens qu'ils mesurent des phénomènes électriques générés naturellement par le corps humain, qui ont l'avantage d'être non invasifs mais dont les possibilités en termes de diagnostic sont limitées.

On connaît également des systèmes de mesure électrophysiologique dits actifs, à base d'impédancemétrie. Le principe de fonctionnement de ces appareils consiste à faire circuler des courants entre différentes électrodes placées sur le corps, et à examiner la manière dont certaines régions du corps atténuent ce courant. En effet ces techniques, à fréquences assez élevées, ont l'inconvénient de dépendre fortement de l'interface peau électrode et en particulier de son effet capacitif. La reproductibilité des mesures entre patients voire sur un même patient est sujette à caution pour ces mesures à haute fréquence. Les mesures en très basses fréquences, elles, peuvent être nocives pour les cellules. Le document US 5 800 350 décrit un exemple d'une telle technique.

La présente invention vise à élargir les possibilités de diagnostic des systèmes de type électrophysiologique, en leur permettant de détecter un certain nombre de maladies, pathologies, terrains pathologiques ou autres troubles détectables habituellement par analyse de sang ou autre fluide corporel.

On observera à ce sujet qu'on connaît déjà des systèmes de diagnostic (par exemple au document US 4 794 934) dans lesquels on applique à des électrodes placées sur les doigts d'une même main des créneaux de tension rectangulaire d'une certaine fréquence, nécessairement élevée pour pourvoir détecter les phénomènes capacitifs au niveau de la peau, et l'on étudie le courant qui circule dans cette partie du corps en réponse à cette tension rectangulaire. Ce système a évolué pour intégrer, en liaison avec la fréquence élevée des créneaux, une analyse de Fourier qui donne la répartition spectrale du courant relevé.

Toutefois, ce système connu a des applications limitées à des détections très localisées (au voisinage des doigts des mains et des pieds), et ne permet d'effectuer des diagnostics que dans les limites des techniques conventionnelles d'acupuncture.

L'invention vise donc à proposer un système de diagnostic non invasif, simple d'emploi ayant une spécificité et une sensibilité équivalentes aux analyses de laboratoires et qui permette de détecter avec une fiabilité améliorée et avec un éventail de possibilités plus large certaines pathologies, certaines prédispositions pathologiques ou certains dysfonctionnements d'organes.
L'invention propose à cet effet un système d'analyse électrophysiologique pour la détection de pathologies, tel que defini dans la revendication 1.

Certains aspects préférés, mais non limitatifs de ce système sont définis dans les revendications dépendantes 2 à 13.

Selon un autre aspect, on propose selon l'invention un procédé d'analyse électrophysiologique tel que défini dans la revendication 14.

Certains aspects préférés, mais non limitatifs de ce procédé sont définis dans les revendications 15 à 22.

Dans une application particulière, on propose selon l'invention un procédé tel que défini dans la revendication 23.

Un aspect préféré mais non limitatif de ce procédé est défini dans la revendication 24.

D'autres aspects, buts et avantages de la présente invention apparaîtront mieux à la lecture de la description détaillée suivante de modes de réalisation préférés de celle-ci, donnée à titre d'exemple non limitatif et faite en référence aux dessins annexés, sur lesquels :
La figure 1 est un schéma-bloc illustrant les différents élément fonctionnels d'un système selon la présente invention,
La figure 2 illustre un exemple de graphique de type radar obtenu avec un système selon la présente invention,
La figure 3 est un schéma-bloc modifié, illustrant l'agencement d'un système selon l'invention en deux parties, et
La figure 4 est un graphique illustrant les relevés de courants obtenus avec un système de la présente invention dans le cadre du diagnostic d'affections coronariennes par test d'effort.

On va maintenant décrire un système de diagnostic de mesures électrophysiologiques selon l'invention. Ce système est fondé sur un principe de fonctionnement actif, en mode de courant continu par opposition aux systèmes d'impédancemétrie de l'art antérieur dont les inconvénients ont été rappelés plus haut.

On observera préliminairement que la Demanderesse a effectué des recherches approfondies sur les comportements électriques des cellules du corps humain et de leur environnement. Elle a ainsi constaté qu'un courant électrique alternatif, surtout en basse fréquence, ne passait que dans l'eau interstitielle (interstitium) séparant les cellules et pratiquement pas à travers l'eau des cellules elles-mêmes. En effet les membranes des cellules ont un effet capacitif suffisant pour atténuer pratiquement complètement le courant alternatif aux basses fréquences.

D'autres recherches sur le comportement des membranes des cellules ont permis de démontrer qu'elles se comportent très différemment selon la nature de l'interstitium, en particulier son pH et son potentiel électrique.

Pour utiliser ces propriétés il est possible de soumettre temporairement ces milieux à des conditions différentes de potentiel et de pH et de mesurer leur réactivité.

Une de ces possibilités va être expliquée ci-dessous.

On rappellera tout d'abord que les ions chlorure ont pour propriété, en présence d'eau et d'une différence de potentiel suffisante et en présence d'un pH inférieur à 7,5, de se transformer en HCLO et en H3O+ en délivrant deux électrons.

En revanche, pour des pH supérieurs à 7,5, on observe la formation d'ions CLO-.

Ces transformations ne peuvent avoir lieu qu'au-delà d'une tension critique qui dépend des concentrations respectives de Cl- et de HCLO et de la valeur du pH. Cette tension est typiquement de 0,8V environ aux conditions physiologiques habituelles.

Il apparaît que cette tension critique s'élève lorsque le pH diminue et lorsque la concentration de HCLO augmente, si bien qu'au cours d'une mesure sur un tissu vivant, un arrêt de cette transformation est atteint plus ou moins rapidement selon la tension initiale appliquée, la valeur initiale du pH et l'évolution des concentrations d'ions H+ et de HCLO.

Ce type de mesure permet donc grâce à l'équation chronoampérométrique de Cottrell, d'avoir accès aux concentrations locales d'ions chlorure mais également de tester la réactivité du tissu, in vivo, à une augmentation locale de l'acidité.

En effet sans la présence de tampons naturels comme les bicarbonates (ou autres), cette transformation d'ions chlorure entraînerait des baisses de pH très fortes, qui se traduiraient elles-mêmes par un arrêt très rapide de la réaction et donc du courant résultant.

Cette réaction offre donc également un moyen indirect pour tester localement l'état de l'équilibre acido-basique de l'être vivant, à savoir la faculté des tampons naturels à jouer leur rôle.

Sachant que l'ion chlorure est l'anion le plus présent dans les compartiments extracellulaires des organismes vivants, il est donc possible d'estimer en temps réel, in vivo, et dans des endroits variés du corps comme les mains, les plantes des pieds, le front, etc. la nature ionique de l'interstitium.

Ces mesures de concentrations de chlorures et de réactivité de l'équilibre acido-basique sont susceptibles de tracer des dysfonctionnements homéostatiques, hormonaux, vasculaires, métaboliques, etc. Ces dysfonctionnements peuvent eux-mêmes correspondre à des pathologies ou prédispositions pathologiques variées notamment :
- vasculaires (hypertension et athérome extensif, affections coronariennes, etc.) ;
- hormonales (hypo- et hyperthyroïdie, etc.)
- métaboliques (diabète, etc.).

L'invention telle qu'on va la décrire maintenant en détail vise à exploiter les techniques électrochimiques de chronoampérométrie ou de voltamétrie pour mesurer les courants issus des réactions électrochimiques précitées et ainsi estimer de façon réaliste les conditions régnant dans l'interstitium, permettant elles-mêmes de décrire l'état physiologique des êtres vivants.

On notera dès à présent que des corrections peuvent être nécessaires en fonction des tensions effectivement appliquées et mesurées sur les électrodes, pour tenir compte des courants inverses susceptibles d'être engendrés si une tension dépassant 0,8 Volt apparaît sur l'électrode distale.

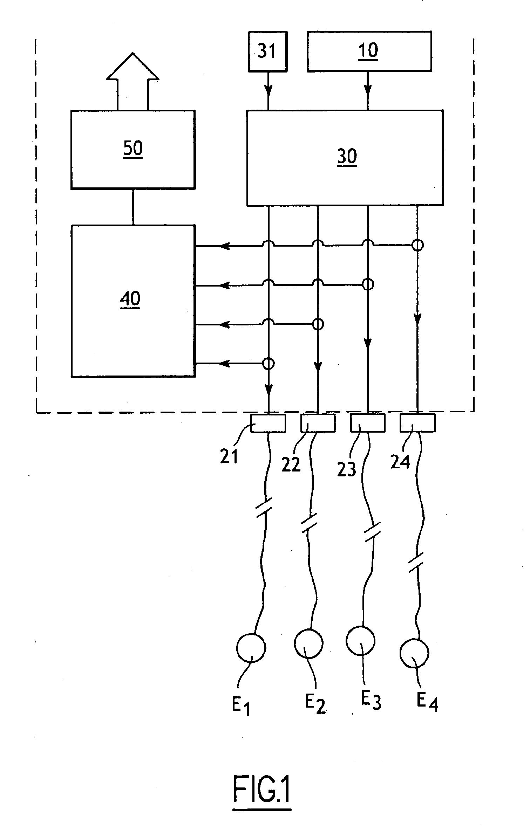

En référence à la figure 1, on a représenté schématiquement un système de diagnostic par mesures électrophysiologiques susceptible d'exploiter les principes précités, qui comprend, dans un boîtier approprié (non illustré), un circuit 10 d'alimentation stabilisée basse tension (typiquement une tension réglable d'environ 1 à 5 volts) susceptible d'être relié comme on le verra dans la suite à des connecteurs 21 à 24 pour une pluralité d'électrodes corporelles (typiquement de 4 à 8).

Dans un exemple d'utilisation, on place quatre électrodes E1 à E4 sur les quatre membres, au niveau des mains et des pieds respectivement. On comprend qu'il existe 6 paires possibles d'électrodes exploitables, à savoir E1-E2, E1-E3, E1-E4, E2-E3, E2-E4 et E3-E4.

Afin que les mesures de courants effectuées grâce aux électrodes reflètent convenablement le comportement électrochimique de l'interstitium, il est important que ces électrodes présentent chacune une surface significative, et de préférence entre environ 1 et 15 cm².

Grâce à un circuit de commutation électronique 30, on applique séquentiellement sur ces paires d'électrodes des créneaux de tension continue, d'une valeur choisie entre 1 et 5 volts, ces créneaux pouvant être positifs ou négatifs par simple inversion de polarité.

On obtient ainsi 12 possibilités de mesure de l'évolution du courant qui traverse le corps entre deux électrodes lorsque le créneau de tension continue est appliqué. Le circuit de commutation est cadencé par une horloge 31, de façon aisément compréhensible par l'homme du métier.

Le système comprend en outre un circuit d'acquisition (échantillonnage et conversion analogique/ numérique) 40 destiné, dès l'application d'un créneau de tension continue, à mémoriser les valeurs prises par le courant selon des intervalles temporels courts prédéterminés.

Par exemple, le système peut engendrer un créneau de tension continue d'une durée comprise entre 0,1 et 5 secondes (typiquement de 3 secondes), et pendant la durée de ce créneau, on échantillonne le courant toutes les millisecondes. Plus généralement, on utilisera une fréquence d'échantillonnage comprise entre 20 et 10.000 échantillons par seconde.

Le système commute alors sur la paire d'électrodes suivante pour renouveler le processus, et ainsi de suite.

Le système comprend également un dispositif 50 de traitement en vue d'analyser des valeurs acquises dans le processus décrit ci-dessus, ce dispositif comprenant typiquement un processeur en association avec une mémoire. Il est relié à des moyens indicateurs (écran d'affichage, imprimante, ou toute autre signalisation) pour l'opérateur.

De préférence, ce dispositif est conçu pour réaliser tout d'abord un ensemble de tests de validation de mesures sur les valeurs de courant recueillies par le circuit d'échantillonnage 40.

Un premier test vise à déterminer si les mesures effectuées sont normales, et à rejeter les mesures contenant des anomalies telles qu'elles ne peuvent être que non significatives.

Ce premier test est réalisé à l'issue de 36 jeux de mesures effectuées, à savoir les 12 mesures décrites plus haut effectuées ici en trois passages. On appelle ici courbe le jeu de mesures du courant obtenues pour chaque créneau de tension continue.

Pour chacune des 36 courbes, le dispositif mémorise les valeurs des deux premiers échantillons relevés et numérisés, et calcule également une moyenne des douze premiers échantillons.

Le dispositif détermine alors si les moyennes calculées pour chacun des deuxième et troisième passages sont, pour chaque courbe, identiques l'une à l'autre à x% près, avec x de préférence compris entre 1 et 10%.

Dans le cas où, pour au moins l'une des courbes, l'écart entre les moyennes est supérieur à cette limite, alors le jeu des 36 courbes est rejeté.

Si le jeu de courbes passe le premier test ci-dessus, le dispositif de traitement 50 réalise un deuxième test de validité consistant à vérifier si les X dernières valeurs (avec X de l'ordre de quelques unités à quelques dizaines) des deuxième et troisième jeux de mesures sont proches l'une de l'autre ave une marge d'erreur donnée, de préférence de l'ordre de 1 à 10% également.

Dans l'affirmative, les mesures sont considérées comme valides.

Bien entendu, toute autre combinaisons des valeurs de courants relevés peuvent être exploitées pour déterminer la validité des mesures.

Dans la négative, l'ensemble de l'équipement est vérifié (positionnement et stabilité des électrodes notamment) et la campagne de mesures est recommencée.

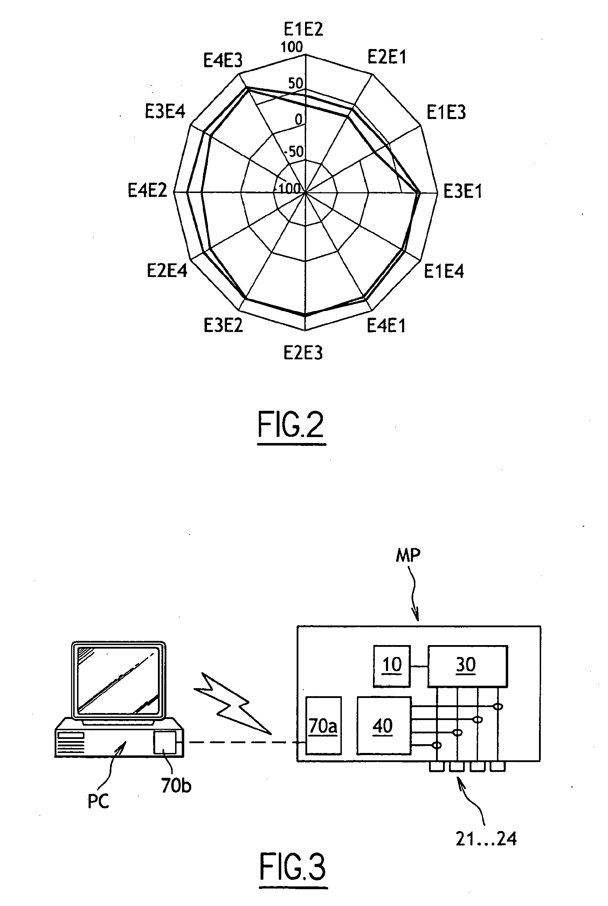

Le dispositif, à partir des mesures validées, réalise alors une transformation plus ou moins complexe destinée à garantir la reproductibilité intrinsèque des données collectées. Cette transformation consiste, dans le présent exemple, à prendre la moyenne, sur les trois secondes que dure ici chaque créneau, de chacune des 12 courbes du dernier (ici troisième) passage, et engendre des informations de sortie pour affichage selon un schéma dit radar, ce schéma incluant par ailleurs, de façon préalablement mémorisée dans le système, des gabarits de mesure correspondant à des patients sains et à des patients atteints. Un exemple d'un tel schéma radar est illustré sur la figure 2 des dessins. Bien entendu, de nombreuses autres transformations sont envisageables, en fonction notamment des caractéristiques du courant et des pathologies recherchées.

Un tel schéma de type radar est bien connu en ergonomie. Il consiste à répartir les résultats des mesures obtenues avec les différentes paires d'électrodes selon un système de coordonnées polaires, une position angulaire donnée étant dédiée à la représentation d'un niveau de signal obtenu (issu d'un traitement approprié) pour une paire d'électrodes donnée telle qu'identifiée sur la figure.

Il a l'avantage de donner une bonne indication visuelle des similitudes et des différences entre des jeux de mesures provenant par exemple de patients différents, ou du même patient à des moments différents, et de faciliter la comparaison avec des gabarits de patients sains et de patients atteints.

Outre l'affichage à destination de l'opérateur, le dispositif de traitement est également apte à réaliser une comparaison chiffrée entre les mesures et les gabarits, par des algorithmes qui sont à la portée de l'homme du métier.

Notamment, si un gabarit de patient sain est dépassé en plus de trois points, ou de plus de X% (par exemple 30%) en un point quelconque, l'opérateur applique au dispositif de traitement une commande de comparaison avec un gabarit lié à une pathologie donnée, et ces comparaisons sont répétées jusqu'à obtention d'un gabarit conforme à la pathologie potentielle.

Bien entendu, de nombreuses autres formes de réalisation et variantes sont possibles. On peut jouer notamment sur le nombre d'électrodes, et recourir par exemple à huit électrodes (quatre pour les extrémités des membres, deux pour le front et deux pour la poitrine).

En fonction notamment des possibilités (puissance de calcul et mémoire) du dispositif de traitement, on peut ajuster la période d'échantillonnage du courant mesuré, celle-ci pouvant descendre par exemple jusqu'à 0,1 ms (fréquence d'échantillonnage de 10 kHz).

On peut aussi prévoir une fréquence d'échantillonnage variable, plus élevée au début des mesures de courant pour un créneau donné et plus faible vers la fin du créneau.

Le dispositif de traitement peut également être conçu pour fournir une information représentative de la pente (dérivée) du signal de courant juste après le début du créneau de tension, et pour calculer le temps qui s'est écoulé entre le début du créneau de tension et l'instant auquel, pendant que le créneau perdure, le courant s'est stabilisé (à x % près, x étant choisi notamment en fonction de la précision de l'échantillonnage).

De telles valeurs, déterminées pour un ensemble donné de paires d'électrodes et de polarité des créneaux, peuvent ensuite être comparées à des valeurs limites, ici encore avec le recours à des schémas radar si cela est approprié.

D'une façon plus générale, différents types d'algorithmes peuvent être mis en oeuvre pour traiter les N lots de courbes (selon le nombre d'électrodes et d'essais successifs) pour valider des seuils de sensibilité et de spécificité plus fins par comparaison avec une courbe de référence ROC (pour « Receiver Operating Characteristic » en terminologie anglo-saxonne) obtenue avec des patients sur lesquels des tests cliniques ont été validés.

Selon une autre caractéristique préférée, le circuit de traitement est apte à engendrer des résultats assortis d'une probabilité, selon des algorithmes connus là encore de l'homme du métier. Si la probabilité n'est pas satisfaisante, on peut relancer une campagne de mesures avec des valeurs de tensions différentes (par exemple croissantes) pour chaque passage sur l'ensemble des paires d'électrodes.

A cet égard, le circuit de commutation 30, et le cas échéant le circuit d'alimentation 10 pour faire varier le niveau de tension, sont avantageusement programmables de manière à pouvoir exécuter différents scénarios de mesure, avec des créneaux de longueur (durée) et le cas échéant de hauteur (niveau de tension) variables.

En référence à la figure 3, on a illustré une forme de réalisation possible d'un système selon l'invention. Ce système est construit autour d'un ordinateur personnel conventionnel PC apte à communiquer avec un module portable MP qui comprend :
- la source d'alimentation 10, si elle n'est pas fournie par le PC ;
- l'ensemble de connecteurs 21 à 24 pour les électrodes corporelles ;
- le circuit de commutation 30 pour appliquer sur les connecteurs précités des créneaux de tension selon un ou plusieurs scénarios déterminés, comme expliqué plus haut ;
- le module d'acquisition 40 capable d'échantillonner et de numériser selon une fréquence déterminée ou plusieurs fréquences déterminées, comme expliqué plus haut, le courant qui circule d'une électrode à une autre pendant que cette paire d'électrode est assujettie au créneau de tension,
- un module de mémoire tampon 60, si nécessaire, pour stocker les mesures de courant ainsi acquises ;
- un module d'interface ou de communication 70a pour communiquer avec le PC, soit par une liaison filaire (série, USB, etc.), soit par une liaison sans fil (Bluetooth®, Wifi®, infrarouge, etc.).
- un jeu d'électrodes E1 à E4 avec leurs câbles.

Dans une telle architecture, le dispositif 50 de traitement des mesures de courant reçues de l'unité portable est constitué par le PC, qui comprend un module d'interface ou de communication 70b assurant la liaison avec le module portable.

Avantageusement, le PC possède des moyens de communication avec un ou plusieurs serveurs publics ou privés à partir desquels peuvent être transférés des gabarits de patients sains ou atteints aux fins de comparaison comme décrit dans ce qui précède, et/ou vers lesquels les informations engendrées par le dispositif de traitement 40 peuvent être envoyées.

A ce sujet, on va maintenant décrire un système de traitement temps réel ou quasi-réel de données cliniques issues de données fournies par un système de diagnostic tel que celui qui a été décrit dans ce qui précède.

Plus précisément, cet aspect de l'invention consiste à exploiter la simplicité et la spécificité des mesures de chronoampérométrie fournies par un ensemble de systèmes de diagnostic pour pouvoir proposer en temps réel ou quasi-réel les informations les plus à jour.

Dans une forme préférée, le processus comprend les étapes essentielles suivantes :
- agrément d'un certain nombre de centres cliniques de référence, équipés du système de diagnostic et reliés par un système de communication approprié avec un serveur central ou des serveurs répartis (systèmes abonnés avec possibilité de paiement pour chaque transaction) ;
- centralisation au niveau du ou des serveurs, de préférence selon une fréquence au moins quotidienne, des mesures effectuées sur des patients pour lesquels on dispose par ailleurs de diagnostics cliniques bien établis et normés, ces mesures étant accompagnées de données de préférence structurées ou encodées représentatives desdits diagnostics cliniques établis ;
- exploitation de la base de données ainsi constituée, avec de préférence les sous-étapes suivantes :
   * validation des données, et plus particulièrement validation statistique et vérification de l'application de lois normales ;
   * analyse de la cohérence des données (par corrélations en particulier) et traitement des écarts trop importants avec enquêtes spécifiques par du personnel compétent sur ces données ;
   * recherche d'algorithmes pertinents sur les données, et plus particulièrement parmi un ensemble d'algorithmes connus et préalablement mémorisés dans le ou les serveurs et basés notamment sur les outils suivants :
      - approche par le théorème de Bayes (réseaux Bayesiens) ;
      - approche par réseaux de neurones multiples à au moins deux couches
      - approche par arbres de décision ;
      - approche par algorithmes génétiques ;
   * comparaison des résultats fournis par ces algorithmes avec les connaissances diagnostiques existantes ;
   * le cas échéant, comparaison des données recueillies avec des données pré-existantes selon d'autres règles telles que des règles de classement ;
   * validation des algorithmes existants, en utilisant chaque nouveau lot de données comme estimateur des valeurs prédictives positives et négatives pour chaque pathologie ou prédisposition pathologique retenue comme pouvant être diagnostiquée ;
   * validation des nouveaux algorithmes identifiés et représentatifs d'une nouvelle connaissance diagnostique sur l'ensemble des données existant précédemment dans la base de données.

De préférence, l'ensemble des processus décrits ci-dessus, du chargement des données à partir des systèmes de diagnostic distants jusqu'aux validations d'algorithmes existants et nouveaux est automatisé, seules les anomalies détectées faisant l'objet d'une signalisation particulière pour examen par des experts compétents.

L'ensemble des processus de collecte et d'analyse précités étant effectués par exemple sur une base au moins quotidienne, ils fournissent ainsi un service mis à jours pratiquement en temps réel avec les nouvelles fonctionnalités diagnostiques identifiées par ces processus.

Selon un aspect avantageux, dans le cas où les systèmes de diagnostic locaux possèdent la capacité de traitement appropriée, ces nouvelles fonctionnalités (nouveaux algorithmes et des informations sur leur rôle diagnostique à l'usage des praticiens) sont avantageusement téléchargées du ou des serveurs vers les systèmes abonnés (avec un éventuel paiement unitaire sécurisé au moment de la transaction) lorsqu'elles ont été identifiées, selon les techniques informatiques classiques de mise à jours de logiciels ou de leurs bibliothèques, plug-ins ou autres modules associés.

De façon non limitative, le système de l'invention peut être utilisé pour le diagnostic de pathologies de type cardiaque, endocrinien, neurologique, etc.

Une application particulière de la présente invention est le diagnostic par test d'effort des patients souffrant d'affections artéro-coronariennes (CAD en terminologie anglo-saxonne pour Coronary Artery Disease).

### Exemple

Dans une population de 95 hommes choisis au hasard, d'âges compris entre 21 et 83 ans et d'une moyenne d'âge de 57 ans, la présente invention a été mise en oeuvre pour détecter des affections angiocoronarographiques au niveau des artères. Les mesures chronoampérométriques effectuées à l'aide du système de la présente invention ont été effectuées avant et après un test d'effort mené selon le protocole de Bruce. La population comprenait des sujets identifiés comme souffrant d'affection, et d'autres sujets identifiés comme sains. La population affectée était par ailleurs décomposée en sujets souffrant de diabète et en sujets sans affection diabétique.

Le système de l'invention comprenait dans ce cas deux électrodes surfaciques prévues au niveau des mains et deux autres électrodes surfaciques prévues sur la tête au niveau des lobes frontaux gauche et droit.

Les courants apparaissant en réponse aux impulsions de tension, révélateurs des réactions électrochimiques conformément à la loi de Cottrell, sont mémorisés. On relève ainsi les courants entre les deux électrodes des mains, et les courants entre les deux électrodes du front.

La durée totale de la collecte des courants mesurés est inférieure à 1 minute.

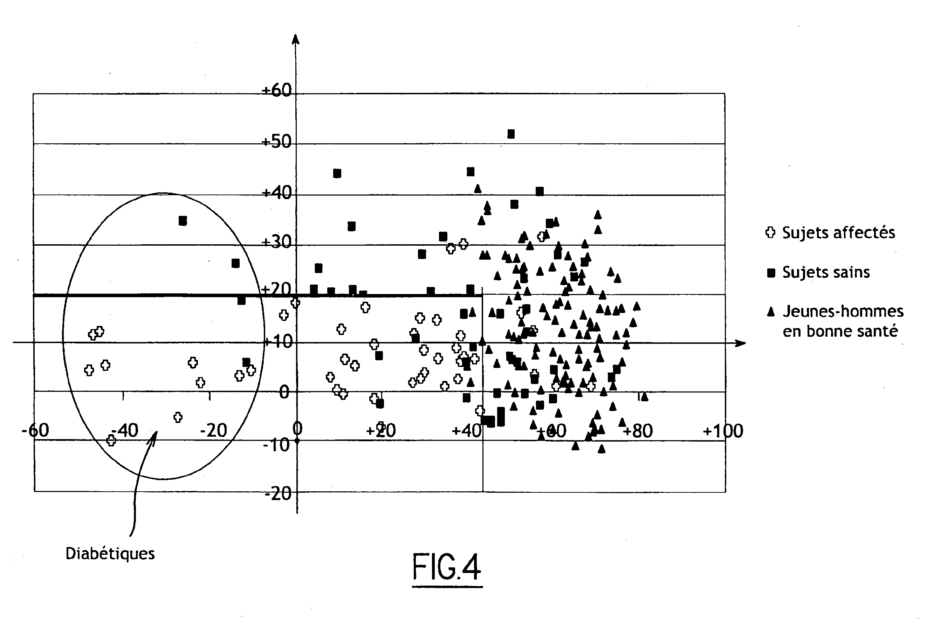

Le graphique illustré sur la figure 4 des dessins montre un ensemble de points dont les coordonnées sont la conductance électrochimique relative obtenue entre les électrodes des mains (abscisse) et entre les électrodes du front (ordonnées) pour les différents sujets.

On notera ici que la conductance électrochimique relative est la différence, positive ou négative, de la conductance électrochimique absolue relevée grâce à la présente invention avant l'effort et après l'effort selon le protocole de Bruce.

Parmi les sujets, on trouve la population précitée de 95 sujets affectés et sains, les mesures correspondant aux sujets sains étant illustrés par des carrés et les mesures correspondant aux sujets ayant une affection étant illustrées par des cercles.

On a également représenté sur la figure 4 les mesures obtenues avec une populations de 150 jeunes hommes en bonne santé, n'ayant normalement aucune affection coronarienne.

Le graphique de la figure 4 montre que le système et le procédé de la présente invention permettent d'obtenir une spécificité de diagnostic d'environ 82% et une sensibilité de diagnostic d'environ 83%, aboutissant à une valeur de prédiction positive de 81% et à une valeur de prédiction négative de 83% (intervalles à 5% de risque inférieurs à 10%). Les seuils correspondants ont été identifiés à environ +43% pour la conductance électrochimique au niveau des mains et à environ +19% pour la conductance électrochimique au niveau du front.

On notera ici que l'âge, l'indice de masse corporelle (BMI), les traitements médicamenteux en cours, le décalage du segment ST et les autres paramètres associés au test d'effort n'avaient aucune influence significative sur les résultats.

On observe également à partir des relevés de la figure 4 que le groupe de 14 sujets préalablement identifiés comme diabétiques est également très bien localisé, avec un seuil de conductance électrochimique relative au niveau des mains inférieure à -6%, ce qui permet d'enrichir la fonction de diagnostic sans opération supplémentaire.

La population des 150 jeunes hommes sains montrée sur la figure 4 confirme la validité des critères identifiés, avec une valeur de prédiction négative supérieure à 90%.

Avantageusement, un système de diagnostic par analyse des courants avant et après l'effort peut être incorporé aux équipements d'effort en salle du commerce tels que vélos d'appartement, tapis roulants pour la course à pied, etc. en permettant au sujet, à l'aide de la simple application d'électrodes sur les poignets et sur le front, d'évaluer directement, à domicile ou en salle de gymnastique et sans nécessité d'un spécialiste, sa prédisposition à des troubles coronariens.

A partir des enseignements qui précèdent, l'homme du métier saura procéder à la mise au point des systèmes de diagnostic pour des pathologies ou prédispositions pathologiques très variées, essentiellement par examen des mesures révélées par le système et le procédé de la présente invention sur des populations de sujets respectivement sains et affectés.

## Revendications

1. Système d'analyse électrophysiologique pour la détection de pathologies, **caractérisé en ce qu'**il comprend :
une pluralité d'électrodes surfaciques (E1-E4) destinées à être positionnées en des endroits distants du corps humain,
des moyens d'alimentation (10, 30) pour appliquer successivement à des paires d'électrodes surfaciques (E1-E4) présentant chacune une surface entre 1 et 15 cm² différentes des créneaux d'une tension essentiellement continue comprise entre environ 1 et 5 volts et ayant des durées comprises entre environ 0,1 seconde et 5 secondes, de manière à engendrer dans les paires d'électrodes pendant chaque créneau un courant d'origine électrochimique qui évolue,
des moyens d'acquisition et de mémorisation (40) pour enregistrer l'évolution du courant circulant dans les paires d'électrodes auxquelles sont appliqués ces créneaux de tension,
des moyens (50) de validation des évolutions de courant acquises par comparaison entre au moins deux évolutions de courant provoquées dans des conditions supposées identiques, et
des moyens de traitement (50) pour comparer des données relatives aux évolutions de courant enregistrées pour plusieurs paires d'électrodes et validées avec des données de référence.

2. Système selon la revendication 1, **caractérisé en ce que** les moyens d'acquisition (40) comprennent des moyens d'échantillonnage du signal de courant à une cadence d'environ 20 à 10000 fois par seconde.

3. Système selon la revendication 1 ou 2, **caractérisé en ce que** les moyens d'alimentation (10, 30) comprennent des moyens pour appliquer à une même paire d'électrodes des créneaux de tension de signes opposés.

4. Système selon l'une des revendications 1 à 3, **caractérisé en ce qu'**il est prévu des moyens pour ajuster les niveaux de tension continue des créneaux.

5. Système selon la revendication 4, **caractérisé en ce qu'**il est prévu des moyens pour ajuster la durée des créneaux.

6. Système selon l'une des revendications 1 à 5, **caractérisé en ce que** les moyens de validation (50) comprennent des moyens pour comparer les évolutions de courant obtenues pour des séries de créneaux de tension appliquées plusieurs fois aux électrodes et pour valider ces évolutions si un écart inférieur à un seuil est constaté.

7. Système selon l'une des revendications 1 à 6, **caractérisé en ce qu'**il comprend en outre des moyens (50) pour engendrer des représentations graphiques des évolutions de courants pour les différentes paires d'électrodes, et des représentations graphiques correspondantes des données de référence.

8. Système selon l'une des revendications 1 à 7, **caractérisé en ce que** les moyens de traitement (50) comprennent des moyens pour déterminer la pente de l'évolution du courant au début de chaque créneau de tension.

9. Système selon l'une des revendications 1 à 8, **caractérisé en ce que** les moyens de traitement (50) comprennent des moyens pour déterminer la durée écoulée entre le début du créneau et l'instant auquel la valeur du courant s'est stabilisée.

10. Système selon l'une des revendications 1 à 9, **caractérisé en ce que** les moyens d'acquisition (40) et au moins une partie des moyens d'alimentation (10, 30) sont prévus dans une unité mobile (MP) dotée d'une pluralité de connecteurs (21, 24) pour les électrodes, **en ce que** les moyens de traitement (50) sont prévus dans un calculateur (PC) séparé de ladite unité mobile, et **en ce qu'**il est prévu un canal de communication (70a, 70b) entre l'unité mobile et le calculateur.

11. Système selon la revendication 10, **caractérisé en ce que** le canal de communication est sans fil.

12. Système de diagnostic dynamique, **caractérisé en ce qu'**il comprend en combinaison :
- des moyens serveurs ;
- une pluralité de systèmes d'analyse selon l'une des revendications 1 à 11 reliés aux moyens serveurs par des canaux de communication ;
- des moyens pour collecter des données relatives aux évolutions de courant mesurées par les systèmes d'analyse sur des patients dont des diagnostics cliniques < sont pré-établis, en association avec des données représentatives desdits diagnostics pré-établis ;
- des moyens de gestion d'algorithmes prévus dans les moyens serveurs et mis en oeuvre de façon périodique en utilisant les nouvelles données collectées pour valider des algorithmes de diagnostic présélectionnés et pour identifier de nouveaux diagnostics.

13. Système selon la revendication 12, **caractérisé en ce qu'**il comprend en outre des moyens pour transférer vers les systèmes d'analyse des algorithmes nouvellement validés ou identifiés en association avec des informations relatives au pathologies ou dispositions pathologiques qu'ils sont capables de détecter.

14. Procédé d'analyse électrophysiologique pour la détection de pathologies, **caractérisé en ce qu'**il comprend les étapes suivantes :
(a) appliquer successivement à une pluralité de paires d'électrodes surfaciques présentant chacune une surface comprise entre 1 et 15 cm² (E1-E4) positionnées en des endroits distants du corps humain, des créneaux d'une tension essentiellement continue comprise entre environ 1 et 5 volts et ayant des durées comprises entre environ 0,1 seconde et 5 secondes, de manière à engendrer dans les paires d'électrodes pendant chaque créneau un courant d'origine électrochimique qui évolue,
(b) enregistrer l'évolution du courant circulant dans les différentes paires d'électrodes auxquelles sont appliqués ces créneaux de tension,
(c) valider des évolutions de courant acquises par comparaison entre au moins deux évolutions de courant provoquées dans des conditions supposées identiques, et
(d) comparer des données relatives aux évolutions de courant telles qu'enregistrées pour plusieurs paires d'électrodes et validées avec des données de référence.

15. Procédé selon la revendication 14, **caractérisé en ce que** l'étape d'enregistrement est mise en oeuvre en échantillonnant le signal de courant à une cadence d'environ 20 à 10000 fois par seconde.

16. Procédé selon la revendication 14 ou 15, **caractérisé en ce que** l'étape d'application de créneaux de tension comprend l'application à une même paire d'électrodes de créneaux de tension de signes opposés.

17. Procédé selon l'une des revendications 14 à 16, **caractérisé en ce qu'**il comprend une étape d'ajustement des niveaux de tension continue des créneaux.

18. Procédé selon l'une des revendications 14 à 17, **caractérisé en ce qu'**il comprend une étape d'ajustement de durée des créneaux.

19. Procédé selon l'une des revendications 14 à 18, **caractérisé en ce que** l'étape de validation comprend la comparaison des évolutions de courant obtenues pour des séries de créneaux de tension appliquées plusieurs fois aux électrodes et la validation de ces évolutions si un écart inférieur à un seuil est constaté.

20. Procédé selon l'une des revendications 14 à 19, **caractérisé en ce qu'**il comprend en outre une étape consistant à engendrer des représentations graphiques des évolutions de courants pour les différentes paires d'électrodes, et des représentations graphiques correspondantes des données de référence.

21. Procédé selon l'une des revendications 14 à 20, **caractérisé en ce qu'**il comprend en outre l'étape consistant à déterminer la pente de l'évolution du courant au début de chaque créneau de tension.

22. Procédé selon l'une des revendications 14 à 21, **caractérisé en ce qu'**il comprend en outre l'étape consistant à déterminer la durée écoulée entre le début du créneau et l'instant auquel la valeur du courant s'est stabilisée.

23. Procédé selon la revendication 14, notamment pour la détection de troubles coronariens par analyse électrophysiologique, **caractérisé en ce que** :
- l'étape a est mise en oeuvre avant l'exercice d'un effort donné par un sujet, les électrodes surfaciques (E1-E4) étant positionnées au moins dans la région des mains et dans la région du front du sujet.
- répéter les étapes (a) et (b) après que le sujet a exercé ledit effort,
- comparer entre elles des données relatives aux évolutions de courant telles qu'enregistrées pour les paires d'électrodes avant et après l'effort, et

24. Procédé selon la revendication 23, **caractérisé en ce qu'**il comprend en outre une étape de comparaison de l'accroissement de la conductance électrochimique, révélé par.les évolutions de courant entre le moment précédent l'effort et le moment suivant l'effort, au moins pour la paire d'électrodes de mains et la paire d'électrodes de front, avec des seuils respectifs.

## Patentansprüche

1. Elektrophysiologisches Analysesystem zum Erfassen von Erkrankungen, **dadurch gekennzeichnet, dass** es umfasst:
eine Mehrzahl von Oberflächenelektroden (E1 - E4), die dazu bestimmt sind, an verschiedenen Stellen des menschlichen Körpers positioniert zu werden,
Versorgungsmittel (10, 30), um an verschiedene Paare von Oberflächenelektroden (E1 - E4), die jeweils eine Fläche zwischen 1 und 15 cm² aufweisen, nacheinander Spannungsimpulse im Wesentlichen einer Gleichspannung zwischen etwa 1 und 5 Volt anzulegen, die eine Zeitdauer zwischen etwa 0,1 Sekunde und 5 Sekunden haben, so dass bei den Elektrodenpaaren bei jedem Impuls ein sich ändernder Strom elektrochemischen Ursprungs erzeugt wird,
Erfassungs- und Speichermittel (40), um den Verlauf des in den Elektrodenpaaren fließenden Stroms aufzuzeichnen, an welche diese Spannungsimpulse angelegt werden,
Mittel (50) zum Validieren der Stromverläufe, die durch Vergleich zwischen zumindest zwei Stromverläufen erfasst werden, die unter als identisch angenommenen Bedingungen entstehen, und
Verarbeitungsmittel (50), um Daten bezogen auf die bei mehreren Elektrodenpaaren aufgezeichneten und validierten Stromverläufe mit Referenzdaten zu vergleichen.

2. System nach Anspruch 1, **dadurch gekennzeichnet, dass** die Erfassungsmittel (40) Mittel zum Abtasten des Stromsignals mit einem Takt von etwa 20 bis 10 000 mal pro Sekunde aufweisen.

3. System nach Anspruch 1 oder 2, **dadurch gekennzeichnet, dass** die Versorgungsmittel (10, 30) Mittel zum Anlegen von Spannungsimpulsen mit entgegengesetzten Vorzeichen an ein gleiches Paar von Elektroden aufweisen.

4. System nach einem der Ansprüche 1 bis 3, **dadurch gekennzeichnet, dass** Mittel zum Abstimmen der Gleichspannungshöhen der Impulse vorgesehen sind.

5. System nach Anspruch 4, **dadurch gekennzeichnet, dass** Mittel zum Abstimmen der Impulsdauer vorgesehen sind.

6. System nach einem der Ansprüche 1 bis 5, **dadurch gekennzeichnet, dass** die Validierungsmittel (50) Mittel aufweisen, um die Stromverläufe, die für Reihen von mehrfach an die Elektroden angelegten Spannungsimpulsen erhalten werden, zu vergleichen und um diese Verläufe bei einer festgestellten Abweichung unter einem Schwellwert zu validieren.

7. System nach einem der Ansprüche 1 bis 6, **dadurch gekennzeichnet, dass** es ferner Mittel (50) zum Erzeugen von graphischen Darstellungen der Stromverläufe für die verschiedenen Elektrodenpaare und von entsprechenden graphischen Darstellungen der Referenzdaten aufweist.

8. System nach einem der Ansprüche 1 bis 7, **dadurch gekennzeichnet, dass** die Verarbeitungsmittel (50) Mittel zum Ermitteln der Steigung des Stromverlaufs zu Beginn eines jeden Spannungsimpulses aufweisen.

9. System nach einem der Ansprüche 1 bis 8, **dadurch gekennzeichnet, dass** die Verarbeitungsmittel (50) Mittel zum Ermitteln der Zeitspanne aufweisen, die zwischen dem Beginn des Impulses und dem Zeitpunkt, an dem der Stromwert sich stabilisiert hat, abgelaufen ist.

10. System nach einem der Ansprüche 1 bis 9, **dadurch gekennzeichnet, dass** die Erfassungsmittel (40) und zumindest ein Teil der Versorgungsmittel (10, 30) in einer beweglichen Einheit (MP) vorgesehen sind, die mit einer Mehrzahl von Verbindern (21, 24) für die Elektroden versehen ist, dass die Verarbeitungsmittel (50) in einem von der beweglichen Einheit getrennten Rechner (PC) vorgesehen sind, und dass ein Kommunikationskanal (70a, 70b) zwischen beweglicher Einheit und Rechner vorgesehen ist.

11. System nach Anspruch 10, **dadurch gekennzeichnet, dass** der Kommunikationskanal drahtlos ist.

12. Dynamisches Diagnosesystem, **dadurch gekennzeichnet, dass** es im Kombination umfasst:
- Server-Mittel;
- eine Mehrzahl von Analysesystemen nach einem der Ansprüche 1 bis 11, die über Kommunikationskanäle mit den Server-Mitteln verbunden sind;
- Mittel zum Sammeln von Daten bezogen auf die Stromverläufe, die von den Analysesystemen an Patienten gemessen werden, deren klinische Diagnosen vorerstellt wurden, in Verbindung mit den für die vorerstellten Diagnosen repräsentativen Daten;
- Mittel zum Anwenden von Algorithmen, die in den Server-Mitteln vorgesehen und periodisch durchgeführt werden, indem die neu gesammelten Daten zum Validieren der vorgewählten Diagnose-Algorithmen und zum Identifizieren neuer Diagnosen verwendet werden.

13. System nach Anspruch 12, **dadurch gekennzeichnet, dass** es ferner Mittel zum Übertragen von neu validierten bzw. identifizierten Algorithmen in Verbindung mit Informationen bezüglich Erkrankungen bzw. pathologischer Dispositionen zu den Analysesystemen, die sie erfassen können, aufweist.

14. Elektrophysiologisches Analyseverfahren zum Erfassen von Erkrankungen, **dadurch gekennzeichnet, dass** es die nachfolgenden Schritte umfasst:
(a) Anlegen von aufeinanderfolgenden Spannungsimpulsen im Wesentlichen einer Gleichspannung zwischen etwa 1 und 5 Volt mit einer Zeitdauer von zwischen etwa 0,1 Sekunde und 5 Sekunden an eine Mehrzahl von Paaren von Oberflächenelektroden (E1 - E4), die jeweils eine Fläche zwischen 1 und 15 cm² aufweisen und an verschiedenen Stellen des menschlichen Körpers positioniert werden,
(b) Aufzeichnen des Verlaufs des in den verschiedenen Elektrodenpaaren fließenden Stroms, an welche diese Spannungsimpulse angelegt werden,
(c) Validieren der Stromverläufe, die durch Vergleich zwischen zumindest zwei Stromverläufen erfasst werden, die unter als identisch angenommenen Bedingungen entstehen, und
(d) Vergleichen von Daten bezogen auf die Stromverläufe, wie sie bei mehreren Elektrodenpaaren aufgezeichnet und validiert werden, mit Referenzdaten.

15. Verfahren nach Anspruch 14, **dadurch gekennzeichnet, dass** der Schritt der Aufzeichnung durch Abtasten des Stromsignals mit einem Takt von etwa 20 bis 10 000 mal pro Sekunde erfolgt.

16. Verfahren nach Anspruch 14 oder 15, **dadurch gekennzeichnet, dass** der Schritt des Anlegens von Spannungsimpulsen das Anlegen von Spannungsimpulsen mit entgegengesetzten Vorzeichen an ein gleiches Paar von Elektroden umfasst.

17. Verfahren nach einem der Ansprüche 14 bis 16, **dadurch gekennzeichnet, dass** es einen Schritt des Abstimmens der Gleichspannungshöhen der Impulse umfasst.

18. Verfahren nach einem der Ansprüche 14 bis 17, **dadurch gekennzeichnet, dass** es einen Schritt des Abstimmens der Zeitdauer der Impulse umfasst.

19. Verfahren nach einem der Ansprüche 14 bis 18, **dadurch gekennzeichnet, dass** der Schritt der Validierung das Vergleichen der Stromverläufe umfasst, die bei Reihen von Spannungsimpulsen erhalten werden, die mehrmals an die Elektroden angelegt werden, sowie die Validierung dieser Verläufe bei Feststellen einer Abweichung unter einem Schwellwert.

20. Verfahren nach einem der Ansprüche 14 bis 19, **dadurch gekennzeichnet, dass** es ferner einen Schritt umfasst, der darin besteht, graphische Darstellungen der Stromverläufe für die verschiedenen Elektrodenpaare und entsprechende graphische Darstellungen der Referenzdaten zu erzeugen.

21. Verfahren nach einem der Ansprüche 14 bis 20, **dadurch gekennzeichnet, dass** es ferner den Schritt umfasst, der darin besteht, die Steigung des Stromverlaufs zu Beginn eines jeden Spannungsimpulses zu ermitteln.

22. Verfahren nach einem der Ansprüche 14 bis 21, **dadurch gekennzeichnet, dass** es ferner den Schritt umfasst, der darin besteht, die Zeitspanne zu ermitteln, die zwischen dem Beginn des Impulses und dem Zeitpunkt, an dem der Stromwert sich stabilisiert hat, abgelaufen ist.

23. Verfahren nach Anspruch 14, insbesondere zum Erfassen von koronaren Beschwerden durch elektrophysiologische Analyse, **dadurch gekennzeichnet, dass**
- der Schritt a vor dem Ausüben einer vorgegebenen Kraft durch eine Person erfolgt, wobei die Oberflächenelektroden (E1 - E4) zumindest in den Bereich der Hände und in den Bereich der Stirn der Person positioniert werden,
- Wiederholen der Schritte (a) und (b), nachdem die Person die genannte Kraft ausgeübt hat,
- Vergleichen der Daten bezüglich der Stromverläufe, wie sie bei den Elektrodenpaaren aufgezeichnet wurden, vor und nach dem Kraftaufwand.

24. Verfahren nach Anspruch 23, **dadurch gekennzeichnet, dass** es ferner einen Schritt des Vergleichens des Anstiegs des elektrochemischen Leitwerts, der von den Stromverläufen zwischen dem dem Kraftaufwand vorausgehenden Zeitpunkt und dem dem Kraftaufwand nachfolgenden Zeitpunkt erhoben wird, zumindest bei dem Hand-Elektrodenpaar und dem Stirn-Elektrodenpaar, mit jeweiligen Schwellwerten umfasst.

## Claims

1. An electrophysiological examination system for the detection of pathologies, **characterised in that** it includes
a plurality of surface electrodes (E1-E4) destined to be positioned at distant parts of the human body,
power means (10, 30) used to apply, in succession to different surface electrode pairs (E1-E4) each having an area between 1 and 15 cm², essentially continuous voltage pulses of between about 1 and 5 volts and with a length of between about 0.1 and 5 seconds, so as to generate in the electrode pairs during each pulse a current of electrochemical origin which changes,
acquisition and storage means (40) to record the current changes flowing in the electrode pairs as these voltage pulses are applied,
means (50) for validation of the changes in the acquired current readings by comparison between at least two current changes provoked in conditions that are assumed to be identical, and
processing means (50) for comparing the data relating to the current changes recorded for several electrode pairs and validated with reference data.

2. The system according to claim 1, **characterised in that** the acquisition means (40) include means for sampling the current signal at a rate of between about 20 and 10,000 times per second.

3. The system according to claim 1 or 2, **characterised in that** the power means (10, 30) include means to apply, to a given electrode pair, voltage pulses of opposite sign.

4. The system according to one of claims 1 to 3, **characterised in that** means are provided to adjust the continuous voltage levels of the pulses.

5. The system according to claim 4, **characterised in that** means are provided to adjust the duration of the pulses.

6. The system according to one of claims 1 to 5, **characterised in that** the validation means (50) include means to compare the current changes obtained for several series of voltage pulses applied several times to the electrodes, and to validate these changes if a difference below a given threshold is observed.

7. The system according to one of claims 1 to 6, **characterised in that** it also includes means (50) to generate graphical representations of the current changes in the different electrode pairs, and corresponding graphical representations of the reference data.

8. The system according to one of claims 1 to 7, **characterised in that** the processing means (50) include means to determine the slope of the current changes at the start of each voltage pulse.

9. The system according to one of claims 1 to 8, **characterised in that** the processing means (50) include means to determine the time that passes between the start of the pulse and the instant at which the value of the current stabilises.

10. The system according to one of claims 1 to 9, **characterised in that** the acquisition means (40) and at least part of the power means (10, 30) are provided in a mobile unit (MP) that is equipped with a plurality of connectors (21, 24) for the electrodes, **in that** the processing means (50) are provided in a computer (PC) that is separate from the said mobile unit, and **in that** a communication channel (70a, 70b) is provided between the mobile unit and the computer.

11. The system according to claim 10, **characterised in that** the communication channel is wireless.

12. A dynamic diagnosis system, **characterised in that** it includes the following in combination:
- server means;
- a plurality of analysis systems according to one of claims 1 to 11, connected to the server means by communication channels;
- means to collect data relating to the current changes measured by the analysis systems on patients whose clinical diagnoses are established beforehand, in association with data representing the said diagnoses prepared beforehand;
- means for the control of algorithms provided in the server means, and executed periodically using the new collected data in order to validate pre-selected diagnosis algorithms and to identify new diagnoses.

13. The system according to claim 12, **characterised in that** it also includes means for the transfer to the analysis systems of the newly validated or identified algorithms, in association with pathologies or pathological predispositions that they are capable of detecting.

14. An electrophysiological examination method for the detection of pathologies, **characterised in that** it includes the following steps:
(a) applying successively to a plurality of pairs of surface electrodes (E1-E4), each having an area between 1 and 15 cm², positioned at distant parts of the human body, essentially continuous voltage pulses of between about 1 and 5 volts and with a length of between about 0.1 and 5 seconds, so as to generate in the electrode pairs during each pulse a current of electrochemical origin which changes,
(b) recording the current changes flowing in the different electrode pairs as these voltage pulses are applied,
(c) validation of the changes in the acquired current readings by comparison between at least two current changes provoked in conditions that are assumed to be identical, and
(d) comparing data relating to the current changes as recorded for several electrode pairs and validated with reference data.

15. The method according to claim 14, **characterised in that** the recording step is executed by sampling the current signal at a rate of between about 20 and 10,000 times per second.

16. The method according to claim 14 or 15, **characterised in that** the step for application of the voltage pulses includes the application, to a given electrode pair, of voltage pulses of opposite sign.

17. The method according to one of claims 14 to 16, **characterised in that** it includes a step for adjustment of the continuous voltage levels of the pulses.

18. The method according to one of claims 14 to 17, **characterised in that** it includes a step for adjustment of the lengths of the pulses.

19. The method according to one of claims 14 to 18, **characterised in that** the validation step includes the comparison of current changes obtained for several series of voltage pulses applied several times to the electrodes, and the validation of these changes if a difference below a given threshold is observed.

20. The method according to one of claims 14 to 19, **characterised in that** it also includes a step that consists of generating graphical representations of the current changes for the different electrode pairs, and corresponding graphical representations of the reference data.

21. The method according to one of claims 14 to 20, **characterised in that** it also includes a step that consists of determining the slope of the current changes at the start of each voltage pulse.

22. The method according to one of claims 14 to 21, **characterised in that** it also includes a step that consists of determining the time that passes between the start of the pulse and the instant at which the value of the current stabilises.

23. The method according to claim 14, in particular for the detection of coronary problems by electrophysiological examination, **characterised in that**:
- step (a) is implemented before the execution of any given exercise by a subject, the surface electrodes (E1-E4) being positioned at least in the region of the hands and in the region of the forehead of the subject,
- repetition of steps (a) and (b) after the subject has performed the said exercise,
- comparison of the data relating to the current changes as recorded for the electrode pairs before and after exercise.

24. The method according to claim 23, **characterised in that** it also includes a step of comparison of the increase in the electrochemical conductance, revealed by the current changes between the moment preceding the exercise and the moment following the exercise, at least for the hand electrode pair and the forehead electrode pair, with respective thresholds.
